# EUROPÄISCHE PATENTSCHRIFT

(11) **EP 3 600 494 B1**
(45) Veröffentlichungstag und Bekanntmachung des Hinweises auf die Patenterteilung: **24.04.2024**
(21) Anmeldenummer: 18714238.5
(22) Anmeldetag: 28.03.2018
(51) Int. Cl.: A61M 5/14, B01L 9/00

(54) **HALTEVORRICHTUNG FÜR MEDIZINISCHE BEHANDLUNGSVORRICHTUNGEN**
HOLDING ARRANGEMENT FOR MEDICAL TREATMENT DEVICES
DISPOSITIF DE MAINTIEN POUR DES DISPOSITIFS DE TRAITEMENT MÉDICAL

(30) Priorität: 30.03.2017 DE 102017106910
(43) Veröffentlichungstag der Anmeldung: 05.02.2020
(73) Patentinhaber: Fresenius Medical Care Deutschland GmbH, 61352 Bad Homburg (DE)
(72) Erfinder: KLEWINGHAUS, Juergen, 61440 Oberursel (DE); SCHROERS, Alexander, 60389 Frankfurt am Main (DE)
(74) Vertreter: Bobbert & Partner Patentanwälte PartmbB
(86) Internationale Anmeldenummer: PCT/EP2018/057935
(87) Internationale Veröffentlichungsnummer: WO 2018/178160

(56) Entgegenhaltungen:
- DE-U1-202004 020 171
- US-A- 2 269 790
- US-A- 4 787 591
- US-A1- 2006 180 727
- US-A1- 2011 147 550

## Beschreibung

Die vorliegende Erfindung betrifft eine Anordnung gemäß Anspruch 1 und eine Behandlungsvorrichtung gemäß Anspruch 14.

Aus der Praxis sind bereits Haltevorrichtungen bekannt, mittels welcher Module wie Filter, Gasaustauscher, Absorber oder dergleichen lösbar an einer Vorrichtung zum Behandeln eines Patienten befestigt werden können.

Die US 2,269,790A offenbart eine Klemme. Die US 4,787,591 A offenbart eine Laborklemme. Die US 2006/0180727 A1 offenbart eine Klemme für eine chemikalische Reagenz. Die US 2011/0147550 A1 offenbart ein Befestigungssystem für einen extrakorporalen Blutkreislauf. Die DE 20 2004 020 171 U1 offenbart eine Tragevorrichtung für medizinische Geräte an Rollstühlen.

Eine Aufgabe der vorliegenden Erfindung ist es, eine Anordnung und eine Behandlungsvorrichtung angegeben werden.

Die erfindungsgemäße Aufgabe wird durch eine Anordnung mit einem medizinischen Modul mit den Merkmalen des Anspruchs 1 und durch eine Behandlungsvorrichtung mit den Merkmalen des Anspruchs 14 gelöst.

Eine erste Haltevorrichtung zum lösbaren Halten wenigstens des medizinischen Moduls wie eines Blutfilters an einer medizinischen Behandlungsvorrichtung, insbesondere an einer Blutbehandlungsvorrichtung, weist wenigstens eine erste Aufnahme und eine zweite Aufnahme auf.

Das medizinische Modul, insbesondere ausgestaltet als Dialysefilter oder Dialysator, ist mit wenigstens einer ersten Haltevorrichtung und mit wenigstens einer zweiten Haltevorrichtung verbunden.

Die erfindungsgemäße Behandlungsvorrichtung weist wenigstens zwei Haltevorrichtungen auf.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Erfindungsgemäße Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Wenn hierin vom "Modul" die Rede ist, so kann hierunter das erste Modul verstanden werden.

Ein Modul ist erfindungsgemäß eine medizinische Funktionseinrichtung, ein Filter, ein Blutfilter, eine Kartusche, ein Gasaustauscher oder ein Adsorber , die bei der Behandlung eines Patienten zum Einsatz kommt. Beschrieben ist zudem ein Adapter und ein Absorber oder eine andere Einrichtung.

Ein Modul kann ein Disposable oder Nicht-Disposable sein.

In einigen Ausführungsformen weist die Haltevorrichtung keine Sperrvorrichtung zum Sperren gegen ein Verdrehen der ersten Aufnahme gegen die zweite Aufnahme auf.

In einigen Ausführungsformen ist das Modul kein Gerät, insbesondere kein Gerät mit elektronischen Bauteilen.

In einigen Ausführungsformen ist das Modul kein Schlauch, insbesondere kein flexibler Schlauch und/oder weist keinen solchen Schlauch auf.

Optional kann das Modul mit einem Schlauch verbunden oder mit einem Schlauch integral gefertigt sein. Dabei wird das Modul jedoch nicht mit seinem - integralen oder verbundenen - Schlauchabschnitt in der Haltevorrichtung gehalten.

In einigen Ausführungsformen ist das Modul nicht schlauchförmig und/oder nicht flexibel.

In manchen Ausführungsformen weist das Modul eine Zylinderform auf. Das Modul kann einen äußeren Rohrabschnitt aufweisen, der an einer oder beiden Stirnflächen mittels Deckel abgeschlossen ist. Die Stirnflächen und/oder Deckel können Durchgangsöffnungen aufweisen, an welchen Schläuche angeordnet sein können.

In einigen Ausführungsformen ist das Modul nicht länger als 40 cm.

In einigen Ausführungsformen ist die Haltevorrichtung nicht mit einer Einrichtung verbunden, die ihrerseits zu ihrem Befestigen an einer Blutbehandlungsvorrichtung ausgestaltet ist. Die Einrichtung kann eine Abdeckung bzw. Abdeckvorrichtung - z. B. für eine Oberfläche einer Behandlungsvorrichtung - sein, die zum Befestigen von Kartuschen, Filtern oder Ähnlichem hieran ausgestaltet und vorbereitet ist. Die Einrichtung kann aus Kunststoff hergestellt sein. Sie kann großflächig sein. Sie kann Abschnitte aufweisen, welche aus einer Kunststofffolie gefertigt sind. Sie kann Durchgangsöffnungen aufweisen zu ihrem Befestigen an oder vor der Oberfläche der Behandlungsvorrichtung.

In einigen Ausführungsformen ist die Haltevorrichtung keine mehrteilige Haltevorrichtung.

In einigen Ausführungsformen weist die Haltevorrichtung keine U-förmigen Elemente, insbesondere keine U-förmigen Rahmenelemente auf.

In einigen Ausführungsformen weist die erste Aufnahme einen Klemmhalter mit Klemmbacken und einem optionalen Schließelement auf oder ist ein solcher Klemmhalter. Dabei sind die Klemmbacken relativ zueinander bewegbar und/oder von einer ersten Stellung zueinander in eine zweite Stellung zueinander bewegbar angeordnet.

In einigen Ausführungsformen ist die zweite Aufnahme ein Befestigungsabschnitt oder weist einen solchen auf. Dieser dient zum, insbesondere lösbaren, Fixieren der Haltevorrichtung an einem Abschnitt der medizinischen Behandlungsvorrichtung, insbesondere an einem Stangenabschnitt der medizinischen Behandlungsvorrichtung.

In einigen Ausführungsformen weist die Haltevorrichtung zusätzlich zur ersten und zur zweiten Aufnahme eine dritte Aufnahme auf.

In einigen Ausführungsformen weisen sowohl die erste Aufnahme als auch die zweite Aufnahme einen Klemmhalter mit

Klemmbacken und einem optionalen Schließelement auf. Dabei sind die Klemmbacken relativ zueinander bewegbar und/oder von einer ersten Stellung zueinander in eine zweite Stellung zueinander bewegbar angeordnet.

In manchen Ausführungsformen weisen sowohl die erste als auch die dritte Aufnahme - und optional auch die zweite Aufnahme - je wenigstens einen Klemmhalter mit Klemmbacken (oder nicht als Backen ausgeformten Klemmabschnitten) und ggf. einem optionalen Schließelement auf. Dabei sind die Klemmbacken, beispielsweise und ohne hierauf beschränkt zu sein unter Mitwirkung des optionalen Schließelements, relativ zueinander bewegbar und/oder von einer ersten Stellung zueinander in eine zweite Stellung zueinander bewegbar angeordnet.

In einigen Ausführungsformen ist zwischen der ersten und der zweiten Aufnahme und/oder zwischen der ersten und der dritten Aufnahme ein Drehabschnitt angeordnet. Dieser ermöglicht eine Drehbewegung der ersten Aufnahme relativ zur zweiten bzw. der ersten Aufnahme relativ zur dritten Aufnahme.

Der Drehabschnitt kann eine Drehung um eine Drehachse, um zwei oder um mehr Drehachsen erlauben. Er kann entsprechend konfiguriert sein.

In einigen Ausführungsformen sind in der Haltevorrichtung die erste Aufnahme und die zweite Aufnahme identisch.

In manchen Ausführungsformen sind in der Haltevorrichtung die erste Aufnahme und die dritte Aufnahme identisch ausgestaltet.

In einigen Ausführungsformen sind die zweite Aufnahme und die dritte Aufnahme identisch ausgestaltet.

In einigen Ausführungsformen sind in der Haltevorrichtung die erste Aufnahme, die zweite Aufnahme und die dritte Aufnahme identisch.

In einigen Ausführungsformen verläuft eine Gerade durch die erste und durch die dritte Aufnahme. Diese verläuft vorzugsweise nicht auch durch die zweite Aufnahme. Alternativ verläuft die Gerade vorzugsweise auch durch die zweite Aufnahme.

In einigen Ausführungsformen ist zwischen der ersten Aufnahme und der dritten Aufnahme ein Abstand, vorzugweise entlang der Geraden. Dieser ist wenigstens so groß wie eine Breite der ersten Aufnahme, vorzugsweise in einer Richtung entlang der Geraden.

In einigen Ausführungsformen weist die Haltevorrichtung eine Stützstruktur zum Abstützen der Haltevorrichtung auf.

In manchen Ausführungsformen ist die erste Aufnahme zwischen der Stützstruktur und der zweiten Aufnahme angeordnet.

In manchen Ausführungsformen ist die Stützstruktur an der ersten Aufnahme und/oder an der zweiten Aufnahme angeordnet.

Die Stützstruktur kann an der zweiten Haltevorrichtung, aber auch an jeder anderen Haltevorrichtung vorgesehen sein.

Erfindungsgemäß sind wenigstens sowohl die erste Haltevorrichtung als auch die zweite Haltevorrichtung mit dem Modul verbunden.

Erfindungsgemäß ist die zweite Haltevorrichtung sowohl mit dem Modul als auch mit wenigstens einem weiteren Modul oder mit einem Adapter zum Aufnehmen eines weiteren Moduls verbunden.

In manchen Ausführungsformen ist die zweite Haltevorrichtung sowohl oberhalb als auch unterhalb der ersten Aufnahme der ersten Haltevorrichtung mit dem Modul verbunden.

In manchen Ausführungsformen ist die zweite Haltevorrichtung angeordnet, um sich mit einer Stützstruktur hiervon gegen die erste Haltevorrichtung abzustützen.

In einigen Ausführungsformen ist die medizinische Behandlungsvorrichtung mittels der ersten Haltevorrichtung mit einem ersten Modul verbunden. Dabei ist das erste Modul mit wenigstens einer zweiten Haltevorrichtung verbunden, wobei die zweite Haltevorrichtung mit einem zweiten Modul oder einem Adapter zum Aufnehmen eines zweiten Moduls verbunden ist. Dabei ist das zweite Modul oder der Adapter mit einer dritten Haltevorrichtung verbunden.

In einigen Ausführungsformen ist die medizinische Behandlungsvorrichtung ausgestaltet als Vorrichtung zur therapeutischen Apherese, als Vorrichtung zur Leberunterstützungstherapie, als Vorrichtung zur Oxygenation oder als Blutbehandlungsvorrichtung, insbesondere als Dialysevorrichtung oder insbesondere als Hämofiltrationsvorrichtung oder als Hämodialysevorrichtung.

In einigen Ausführungsformen sind wenigstens eine oder alle der Haltevorrichtungen zwischen einem Abschnitt der Behandlungsvorrichtung und einem Modul oder zwischen einem jeweils ersten Modul und einem jeweils zweiten Modul (oder Adapter für ein jeweils zweites Modul) angeordnet. Dabei ist die Haltevorrichtung nicht mit weiteren Strukturen als den Vorgenannten verbunden und/oder wird nicht durch solche gestützt.

In manchen Ausführungsformen sind mehr als drei Module mittels Haltevorrichtungen, vorzugsweise in Reihe oder als Kaskade, mit der Behandlungsvorrichtung verbunden.

In einigen Ausführungsformen sind mehr als drei Haltevorrichtungen vorgesehen.

In manchen Ausführungsformen ist eine Aufnahme ein Befestigungsabschnitt.

In einigen Ausführungsformen ist ein Aufnehmen ein Halten oder Befestigen oder Verbinden.

In manchen Ausführungsformen weist die Haltevorrichtung wenigstens einen Verbindungsabschnitt auf, mittels welchem eine oder mehrere Aufnahmen an der restlichen Haltevorrichtung gehalten werden oder miteinander verbunden sind.

In manchen Ausführungsformen weist der Verbindungsabschnitt oder ein anderer Abschnitt der Haltevorrichtung eine Einrichtung auf, mittels welcher eine oder mehrere Aufnahmen lösbar, vorzugsweise per Hand und/oder ohne Werkzeug, mit weiteren Abschnitten der Haltevorrichtung verbunden sind.

Eine solche Einrichtung kann ein Rastverschluss, ein Schnappverschluss, ein Klemmverschluss, ein Klickverschluss oder ein Verbinder oder dergleichen sein.

In manchen Ausführungsformen ist wenigstens eine Aufnahme einer Haltevorrichtung ausgestaltet und/oder wird verwendet, um an wenigstens einer Aufnahme einer zweiten Haltevorrichtung, insbesondere lösbar, befestigt zu werden. Die beiden Aufnahmen, von denen eine von der anderen aufgenommen wird, können aufeinander abgestimmt ausgestaltet sein.

Manche oder alle erfindungsgemäßen Ausführungsformen können einen, mehrere oder alle der oben und/oder im Folgenden genannten Vorteile aufweisen.

Von Vorteil kann sein, dass mittels der vorliegenden Erfindung eine größere Freiheit geschaffen werden kann, um Module, die bei der Behandlung eines Patienten benötigt werden, mit der zur Behandlung verwendeten Behandlungsvorrichtung lösbar zu verbinden. Module, für die keine Aufnahme an der Behandlungsvorrichtung vorgesehen wurde oder aus Platzgründen keine Aufnahme vorgesehen werden konnte, können mittels der vorliegenden Erfindung dennoch - indirekt - an der Behandlungsvorrichtung befestigt werden.

Dabei können vorteilhaft auch mehrere Module in Kaskadenanordnung indirekt an der Behandlungsvorrichtung befestigt werden.

Die erforderliche oder gewünschte Stabilität der lösbaren Befestigung von Modulen an der Behandlungsvorrichtung kann mittels der Stützabschnitte vorteilhaft gewährleistet werden.

Sind mehrere der verwendeten Haltevorrichtungen identisch zueinander, so können Vorteile u. a. bei der Beschaffung erzielbar sein.

Ein Vorteil der Zerlegbarkeit der Haltevorrichtung etwa mittels des Rastverschlusses, Schnappverschlusses, usw. kann darin bestehen, dass z. B. eine beliebige erste Aufnahme mit einer beliebigen zweiten Aufnahme per Hand und/oder eines einfachen Mechanismus zu einer Haltevorrichtung verbunden werden können. Die Haltevorrichtung kann damit auf einfache Weise auf die konkreten, momentanen und ggf. wechselnden Bedürfnisse des Benutzers angepasst werden.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt zwei Haltevorrichtungen in einer ersten Ausführungsform, direkt bzw. indirekt verbunden mit einer Behandlungsvorrichtung;
- Fig. 2: zeigt eine Haltevorrichtung von schräg oben (also mit Blick auf die Oberseite);
- Fig. 3: zeigt die aus Fig. 1 bekannte Anordnung mit einer Abwandlung;
- Fig. 4: zeigt eine der zu Fig. 3 angesprochenen erfindungsgemäßen Ausgestaltungen;
- Fig. 4a: zeigt eine beispielhafte Ausgestaltung der Haltevorrichtung mit zwei Klemmhaltern;
- Fig. 5: zeigt ausgehend von der Darstellung der Fig. 4 einen zwischen einer ersten Aufnahme und einer zweiten Aufnahme der zweiten Haltevorrichtung angeordneten Drehabschnitt;
- Fig. 6: zeigt die Haltevorrichtung in einer weiteren Ausführungsform mit einer weiteren, dritten Aufnahme;
- Fig. 7: zeigt eine weitere Ausführungsform der Haltevorrichtung mit einer Stützstruktur;
- Fig. 8: zeigt eine zu Fig. 7 ergänzende oder alternative Ausgestaltung, in welcher sich die Stützstruktur seitlich gegen die erste Haltevorrichtung abstützt;
- Fig. 9: zeigt eine zu Fig. 7 und/oder Fig. 8 ergänzende oder alternative Ausgestaltung, in welcher sich die Stützstruktur von oben gegen die erste Haltevorrichtung abstützt;
- Fig. 10: zeigt eine weitere Ausführungsform der vorliegenden Erfindung;
- Fig. 11: zeigt eine Kaskadenanordnung aus beispielsweise drei Haltevorrichtungen, die in Reihe angeordnet sind;
- Fig. 11a: zeigt eine exemplarische Ausgestaltung der zweiten oder dritten Haltevorrichtung, wie sie in Fig. 11 schematisch gezeigt sind; und
- Fig. 12: zeigt weitere Ausgestaltung der Haltevorrichtung.

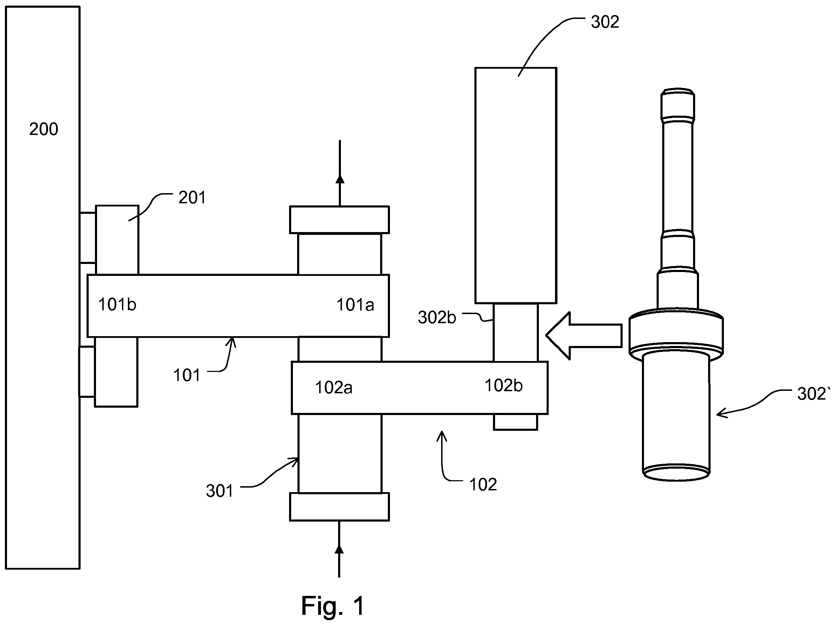

**Fig. 1** zeigt eine erste Ausführungsform der Haltevorrichtung, verbunden mit einer Behandlungsvorrichtung 200.

Die Haltevorrichtung ist hier einmal als erste Haltevorrichtung 101 und einmal als zweite Haltevorrichtung 102 gezeigt. Die erste Haltevorrichtung 101 weist eine erste Aufnahme 101a und eine zweite Aufnahme 101b auf. Die zweite Haltevorrichtung 102 weist eine erste Aufnahme 102a und eine zweite Aufnahme 102b auf.

Die Behandlungsvorrichtung 200 weist einen Stangenabschnitt 201 auf, mit welchem die erste Haltevorrichtung 101 mittels ihrer zweiten Aufnahme 101b lösbar verbunden ist. Mittels ihrer ersten Aufnahme 101a ist die erste Haltevorrichtung 101 mit einem ersten Modul 301, das z. B. ein Dialysator oder Blutfilter sein kann, lösbar verbunden.

Das erste Modul 301 ist wiederum mittels der ersten Aufnahme 102a der zweiten Haltevorrichtung 102 lösbar verbunden. Das erste Modul 301 wird auf diese Weise als Endpunkt für die erste Haltevorrichtung 101 genutzt und durch diese an der Behandlungsvorrichtung 200 gehalten. Zugleich dient das erste Modul 301 seinerseits als Stütze oder Ausgangspunkt für die zweite Haltevorrichtung 102, die auf diese Weise nicht direkt, sondern indirekt über das erste Modul 301, mit der Behandlungsvorrichtung 200 lösbar verbunden ist.

Die zweite Haltevorrichtung 102 weist zusätzlich zu ihrer ersten Aufnahme 102a, mit welcher sie am ersten Modul 301 gehalten wird, eine zweite Aufnahme 102b auf.

Die zweite Aufnahme 102b der zweiten Haltevorrichtung 102 dient im Beispiel der Fig. 1 der Aufnahme eines Gasaustauschers als Beispiel eines zweiten Moduls 302. Das zweite Modul 302 könnte allerdings auch jeder andere Gegenstand sein und ist nicht auf einen Gasaustauscher festgelegt. Das zweite Modul 302 kann wie in Fig. 1 optional die Form einer Kartusche haben.

Das Bezugszeichen 302 kann statt des zweiten Moduls alternativ beispielsweise einen Adapter bezeichnen, auf den ein zweites Modul 302 aufgesteckt werden kann. Für das bessere Verständnis ist in Fig. 1 neben dem schematisch gezeigten zweiten Modul 302 ein Adapter 302' gezeigt, der als zweites Modul 302 zum Einsatz kommen kann und seinerseits der Aufnahme weitere Komponenten, wie etwa einem Gasaustauscher, in den er eingesteckt werden kann, dienen kann.

Die ersten Aufnahmen 101a und 102a einerseits und/oder die zweiten Aufnahmen 101b und 102b andererseits können optional jeweils identisch zueinander sein. Dabei können die erste Haltevorrichtung 101 und die zweite Haltevorrichtung 102 insgesamt identisch zueinander sein.

Der Abschnitt 302b, welcher in der zweiten Aufnahme 102b steckt, kann vorteilhafter Weise denselben oder einen ähnlichen Durchmesser haben wie der Stangenabschnitt 201. Ist dies der Fall, so können die erste Haltevorrichtung 101 und die zweite Haltevorrichtung 102 erst recht identisch ausgestaltet sein.

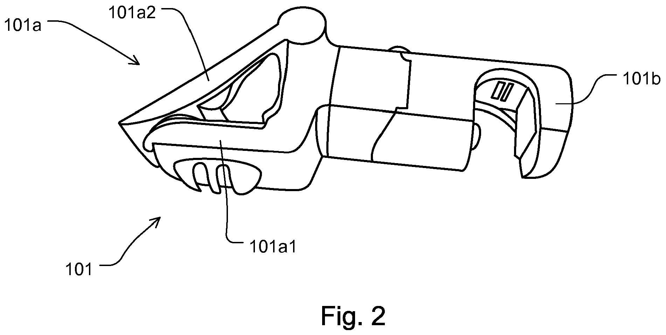

**Fig. 2** zeigt eine erste Haltevorrichtung 101 entsprechend der vorliegenden Erfindung.

Die erste Aufnahme 101a ist als Klemmhalter mit Klemmbacken 101a1 und 101a2 ausgestaltet. Ein Schließelement in Gestalt einer Feder, einer Schraubverbindung, eines Rastmechanismus oder dergleichen ist vorgesehen, nicht aber in Fig. 2 gezeigt.

Die zweite Aufnahme 101b ist optional zur Aufnahme des Stangenabschnitts 201 ausgestaltet. Sie ist hier exemplarisch kein Klemmhalter, könnte optional aber ebenfalls ein solcher sein. Ebenso könnte auch die erste Aufnahme 101a wie die zweite Aufnahme 101b, also nicht als Klemmhalter, ausgestaltet sein.

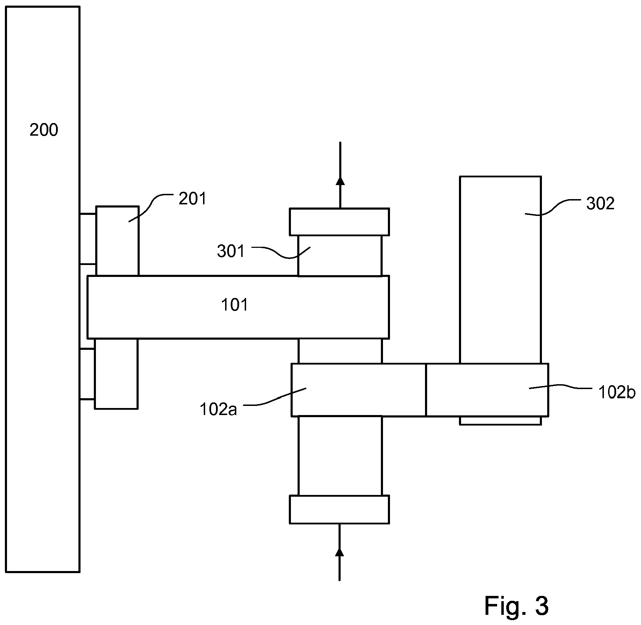

**Fig. 3** zeigt die aus Fig. 1 bekannte erfindungsgemäße Anordnung, mit folgendem Unterschied:
Die zweite Aufnahme 102b der zweiten Haltevorrichtung 102 ist als Adapter ausgestaltet, der der Aufnahme einer weiteren Komponente, eines weiteren Moduls 302, eines (ggf. weiteren) Filters, eines Gasaustauschers, eines Absorbers, und dergleichen dienen kann. Zu diesem Zweck kann die zweite Aufnahme 102b der zweiten Haltevorrichtung 102 wie die erste Aufnahme 102a, mit welcher die zweite Haltevorrichtung 102 am ersten Modul 301 verbunden ist, ausgestaltet sein.

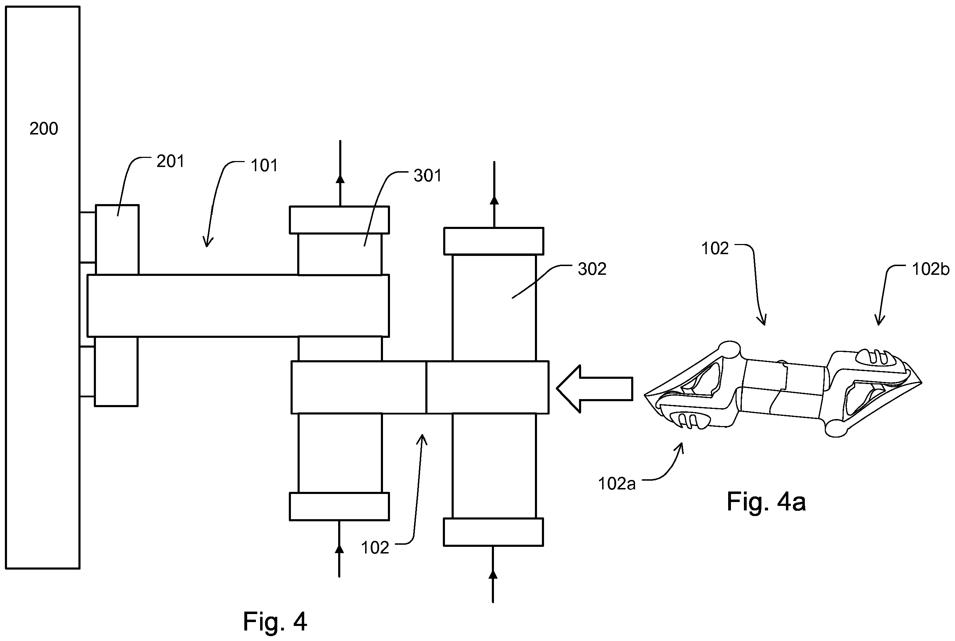

**Fig. 4** zeigt eine der zu Fig. 3 angesprochenen Ausgestaltungen. **Fig. 4a** zeigt eine mögliche Ausgestaltung der zweiten Haltevorrichtung 102, welche hier exemplarisch zwei Klemmhalter, die optional identisch sein können, als erste Aufnahme 102a bzw. zweite Aufnahme 102b aufweist.

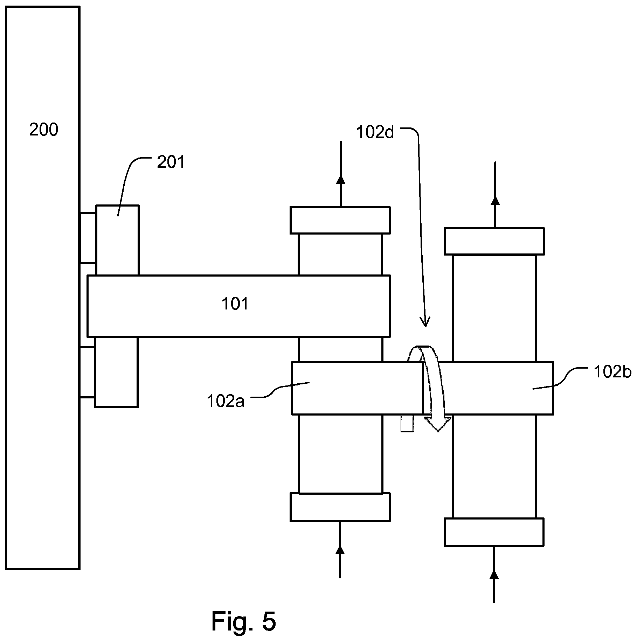

**Fig. 5** zeigt, ausgehend von der Darstellung der Fig. 4 - ohne hierauf beschränkt zu sein-, dass zwischen der ersten Aufnahme 102a und der zweiten Aufnahme 102b der zweiten Haltevorrichtung 102 ein Drehabschnitt 102d vorgesehen ist.

Mittels des Drehabschnitts 102d können die erste Aufnahme 102a und die zweite Aufnahme 102b der zweiten Haltevorrichtung 102 relativ zueinander verdreht werden. Dies kann vorteilhaft die Einsatzmöglichkeiten der zweiten Haltevorrichtung 102 erweitern. Die Drehachse der mittels des Drehabschnitts 102d möglichen Drehung kann in einer beispielhaften Ausführungsform durch die, oder parallel zur, Längsrichtung der zweiten Haltevorrichtung 102 verlaufen.

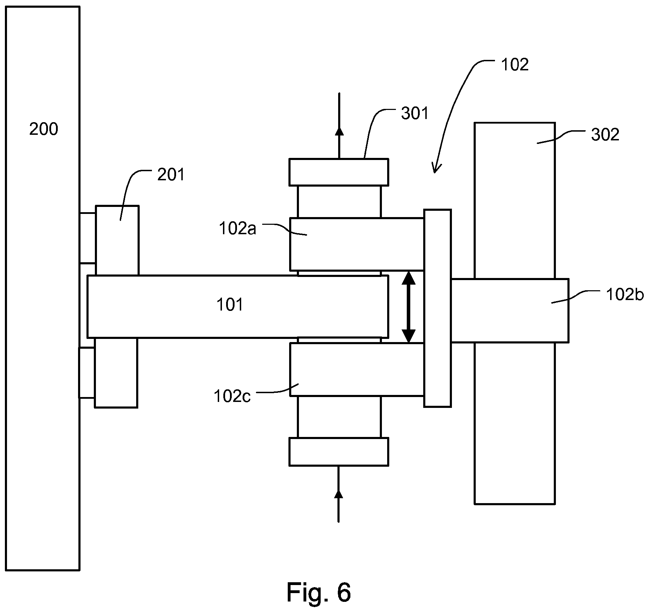

**Fig. 6** zeigt, ausgehend von der Darstellung der Fig. 5 ohne hierauf beschränkt zu sein, dass die zweite Haltevorrichtung 102 zusätzlich zur ersten Aufnahme 102a und zweiten Aufnahme 102b eine dritte Aufnahme 102c aufweisen kann.

Die erste Aufnahme 102a und die dritte Aufnahme 102c sind hier exemplarisch identisch. Beide sind lösbar mit dem ersten Modul 301 verbunden oder nehmen dieses auf. Gemeinsam erhöhen sie nicht zuletzt aufgrund des Abstandes zwischen ihnen die Stabilität der Verbindung der zweiten Haltevorrichtung 102 mit dem mit ihr mittels der zweiten Aufnahme 102b verbundenen zweiten Modul 302.

Der Abstand, der zwischen der ersten Aufnahme 102a und der dritten Aufnahme 102c vorgesehen ist und in Fig. 6 mittels Doppelpfeils bezeichnet ist, ist optional wenigstens so groß, wie die erste Aufnahme 101a der ersten Haltevorrichtung 101 breit ist.

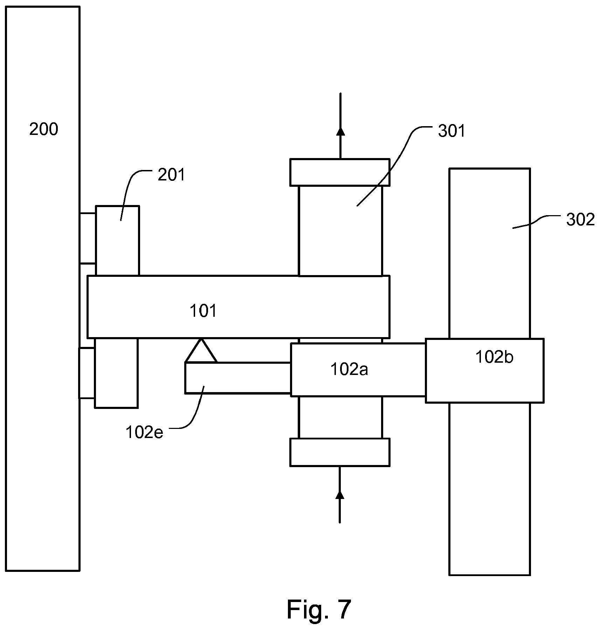

Eine erhöhte Stabilität kann auch durch die in **Fig. 7** gezeigte zweite Haltevorrichtung 102 erzielt werden, die ergänzend zu der in Fig. 6 gezeigten dritten Aufnahme 102c eine Stützstruktur 102e aufweist.

Die Stützstruktur 102e ist im Beispiel der Fig. 7 unterhalb der ersten Haltevorrichtung 101 angeordnet und kann sich gegen diese von unten abstützen.

Wann immer hierin von einer Stützstruktur die Rede ist, so kann ein zum Abstützen eigens vorgesehener oder konfigurierter Abschnitt gemeint sein. Dieser kann optional als Vorsprung ausgestaltet sein. Er kann optional angeordnet sein, um im Bereich einer durch eine obere Fläche der zweiten Haltevorrichtung 102 oder einer ihrer Aufnahmen verlaufenden Ebene zu enden, wie in Fig. 7 gezeigt.

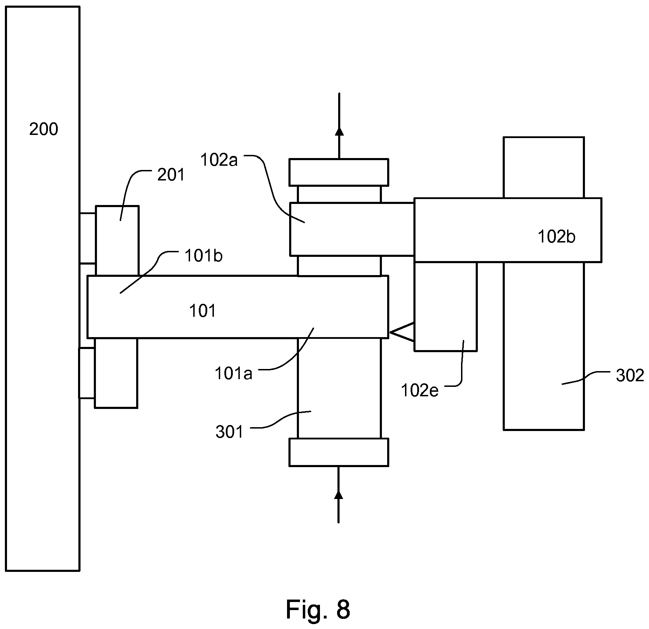

**Fig. 8** zeigt eine zu Fig. 7 ergänzende oder alternative Ausgestaltung, in welcher sich die Stützstruktur 102e seitlich gegen die erste Haltevorrichtung 101 abstützt, etwa gegen die erste Aufnahme 101a. Sie könnte sich alternativ auch gegen das erste Modul 301 oder eine andere Struktur abstützen.

In der exemplarischen Ausführungsform der Fig. 8 steht die Stützstruktur im Winkel, hier optional im rechten Winkel, zur Längsrichtung der zweiten Haltevorrichtung 102, zur ersten Aufnahme 102a oder zur zweiten Aufnahme 102b der zweiten Haltevorrichtung 102.

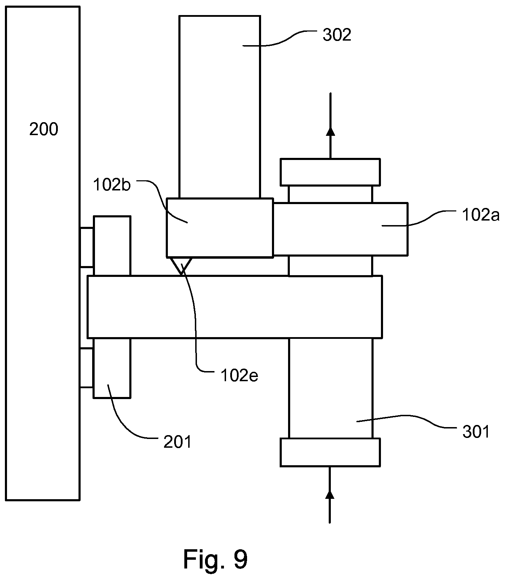

**Fig. 9** zeigt eine zu Fig. 7 und/oder Fig. 8 ergänzende oder alternative Ausgestaltung. In Fig. 9 stützt sich die Stützstruktur 102e von oben gegen die erste Haltevorrichtung 101 ab, etwa gegen die erste Aufnahme 101a, die zweite Aufnahme 101b oder einen Verbindungsabschnitt zwischen erster Aufnahme 101a und zweiter Aufnahme 101b.

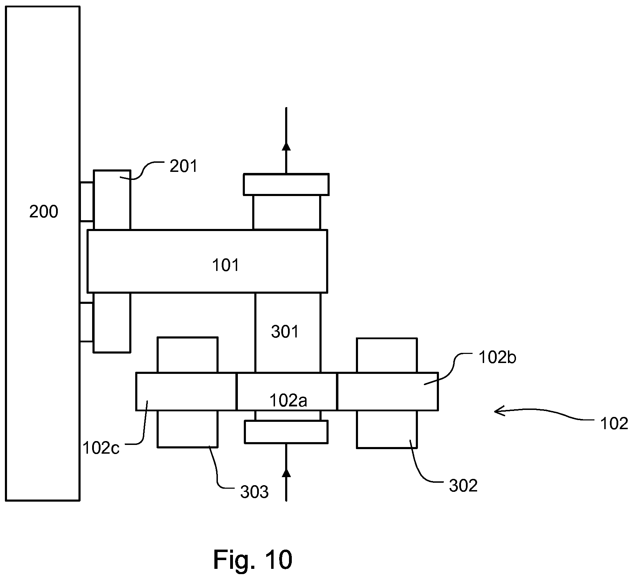

**Fig. 10** zeigt eine weitere Ausführungsform der vorliegenden Erfindung.

Das erste Modul 301 ist wiederum mit einer ersten Haltevorrichtung 101 und mit einer zweiten Haltevorrichtung 102 verbunden. Die zweite Haltevorrichtung 102 weist zusätzlich zur ersten Aufnahme 102a, mit welcher sie mit dem ersten Modul 301 lösbar verbunden ist, und zusätzlich zu der aus den vorangegangenen Figuren bekannten zweiten Aufnahme 102b ferner eine dritte Aufnahme 102c auf. Anders als in Fig. 6, in welcher die erste Aufnahme 102a wie die dritte Aufnahme 102c der Aufnahme ein und desselben ersten Moduls 301 dienen und daher identisch ausgestaltet sein können, gezeigt, ist die dritte Aufnahme 102c exemplarisch jedoch nicht wie die erste Aufnahme 102a ausgestaltet, sondern optional eher oder exakt wie die zweite Aufnahme 102b der Fig. 6 oder Fig. 7, nämlich beispielsweise vorgesehen und konfiguriert zum Aufnehmen eines weiteren Moduls 303.

Alternativ könnte die erste Aufnahme 102a auch hier wie die dritte Aufnahme 102c ausgestaltet sein.

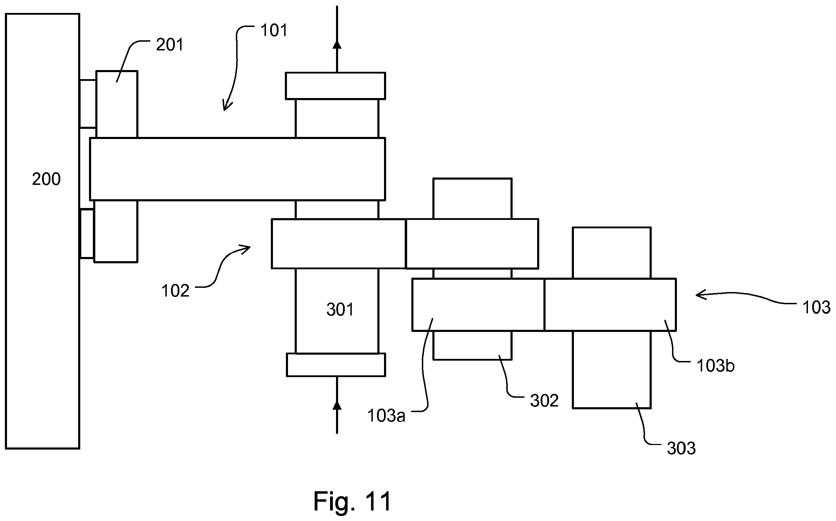

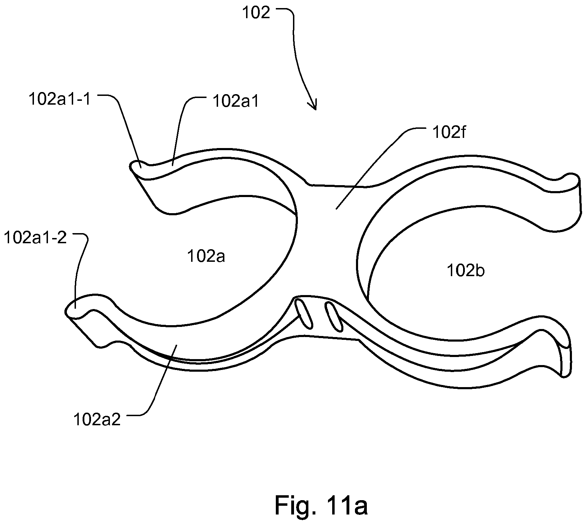

**Fig. 11** zeigt eine Kaskadenanordnung aus beispielsweise drei Haltevorrichtungen 101, 102 und 103 (es könnten alternativ mehr als drei Haltevorrichtungen sein), die in Reihe angeordnet sind. In Reihe bedeutet hier, dass die erste Haltevorrichtung 101 das erste Modul 301 hält, das erste Modul 301, z. B. ein Blutfilter, die zweite

Haltevorrichtung 102 trägt, die zweite Haltevorrichtung 102 ein zweites Modul 302, z. B. einen Gasadsorber, trägt, das zweite Modul 302 seinerseits die dritte Haltevorrichtung 103 mit erster Aufnahme 103a und zweiter Aufnahme 103b trägt und die dritte Haltevorrichtung 103 schließlich ein wiederum weiteres Modul 303 trägt.

Fig. 11a zeigt eine exemplarische Ausgestaltung der zweiten Haltevorrichtung 102, wie sie in Fig. 11 schematisch gezeigt ist. Die zweite Haltevorrichtung 102 kann dabei gleich oder identisch zur dritten Haltevorrichtung 103 der Fig. 11 sein.

Die Haltevorrichtung 102 der Fig. 11a weist eine erste Aufnahme 102a und eine zweite, zur ersten Aufnahme 102a gleiche oder identische Aufnahme 102b auf. Sie stehen miteinander mittels des zwischen ihnen gelegenen Verbindungsabschnitts 102f in Verbindung.

Sowohl die erste Aufnahme 102a als auch die zweite Aufnahme 102b sind als Klemmabschnitte ausgestaltet.

Hierzu weist die erste Aufnahme 102a eine erste Klemmbacke 102a1 und eine zweite Klemmbacke 102a2 auf.

Eines Schließelements bedarf es bei dieser Ausgestaltung nicht, da die Klemmbacken 101a1, 101a2 beide elastisch sind. Sie weichen auseinander, wenn die erste Aufnahme 102a z. B. auf einen Blutfilter aufgeschoben wird, und sie bewegen sich zumindest mit ihren freien Enden 102a1-1 und 102a1-2 wieder aufeinander zu, sobald der Blutfilter mit seinem breitesten Durchmesser an den freien Enden vorbeigeschoben wurde.

Zwar weist die erste Aufnahme 102a der Fig. 11a mit der ersten Klemmbacke 102a1 und der zweiten Klemmbacke 102a2 zwei elastische Abschnitte auf. Es mag im Einzelfall jedoch genügen, nur eine der beiden vorstehend genannten Klemmbacken elastisch auszugestalten.

Eines oder beide der freien Enden 102a1-1, 102a1-2 können wie in Fig. 11a gezeigt optional vom übrigen Teil der ersten bzw. zweiten Klemmbacke 102a1, 102a2 wegstehen. Sie können optional jeweils unter einer zur Krümmung des Hauptabschnitts der Klemmbacke gegenläufigen Krümmung ausgestaltet sein. Letzteres kann das Einschieben z. B. eines Blutfilters in die erste Aufnahme 102a begünstigen. Zudem können die freien Enden 102a1-1, 102a1-2 als Griff für die Betätigung durch den Benutzer dienen.

Wie vorstehend ausgeführt kann die zweite Aufnahme 102b wie die erste Aufnahme 102a ausgestaltet sein.

Die Haltevorrichtung 102 kann aus der ersten Aufnahme 102a, der zweiten Aufnahme 102b und dem Verbindungsabschnitts 102f bestehen.

Der Verbindungsabschnitt 102f der Fig. 11a kann - wie auch Verbindungsabschnitte anderer Ausführungsformen - zwei oder mehrteilig sein und eine entsprechende Einrichtung aufweisen, mittels welcher sie lösbar, vorzugsweise per Hand und/oder ohne Werkzeug, verbunden sind. Eine solche Einrichtung kann ein Rastverschluss, ein Schnappverschluss, ein Klemmverschluss, ein Klickverschluss oder dergleichen sein.

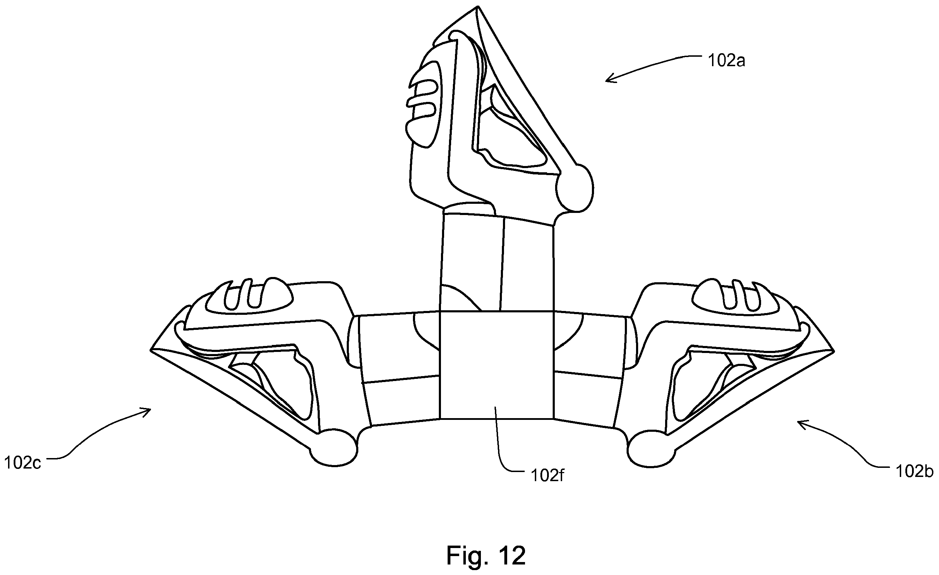

**Fig. 12** zeigt eine weitere Ausgestaltung der Haltevorrichtung, z. B. der zweiten Haltevorrichtung 102, welche drei Klemmhalter, die optional identisch sein können, als erste Aufnahme 102a, zweite Aufnahme 102b und dritte Aufnahme 102c aufweist.

Die drei Aufnahmen 102a, 102b, 102c können wie in Fig. 12 gezeigt durch einen Verbindungsabschnitt 102f miteinander verbunden sein. Jedoch kann der Verbindungsabschnitt 102f seinerseits mit einer Funktion ausgestattet sein; so kann er als Aufnahme ausgestaltet sein, die wiederum konfiguriert ist, einen Adapter aufzunehmen.

Statt drei Klemmhaltern kann die Haltevorrichtung der Fig. 12 auch mehr Klemmhalter aufweisen.

Statt nur Klemmhalter aufzuweisen, könnte eine oder mehrere der Aufnahmen auch anders ausgestaltet sein, etwa wie jene zweite Aufnahme 101b der vorangegangenen Figur 2.

Die Haltevorrichtung der Fig. 12 kann z. B. in der Anordnung der Fig. 10 Verwendung finden.

Der Verbindungsabschnitt 102f kann, wie auch jener der Fig. 11a, eine Einrichtung aufweisen, welche ihn oder einzelne Aufnahmen 101a, 102a, usw. lösbar zusammenhält.

### Bezugszeichenliste

- 101: erste Haltevorrichtung
- 101a: erste Aufnahme
- 101a1: Klemmbacke, erste Klemmbacke
- 101a2: Klemmbacke, zweite Klemmbacke
- 101b: zweite Aufnahme

- 102: zweite Haltevorrichtung
- 102a: erste Aufnahme
- 102a1: Klemmbacke, erste Klemmbacke
- 102a2: Klemmbacke, zweite Klemmbacke
- 102a1-1: freies Ende
- 102a1-2: freies Ende
- 102b: zweite Aufnahme
- 102c: dritte Aufnahme
- 102d: Drehabschnitt
- 102e: Stützstruktur
- 102f: Verbindungsabschnitt

- 103: dritte Haltevorrichtung
- 103a: erste Aufnahme
- 103b: zweite Aufnahme

- 200: Behandlungsvorrichtung
- 201: Stangenabschnitt

- 301: erstes Modul
- 302: zweites Modul, oder Adapter für ein zweites Modul
- 302b: Abschnitt des zweiten Moduls oder Adapters
- 302': Adapter
- 303: weiteres Modul, oder Adapter für ein weiteres Modul

## Patentansprüche

1. Anordnung, aufweisend ein medizinisches Modul (301), ausgestaltet als ein Filter, ein Blutfilter, ein Dialysator, eine Kartusche, ein Gasaustauscher oder ein Adsorber, mit wenigstens einer ersten Haltevorrichtung (101) zum lösbaren Halten des Moduls (301) an einer medizinischen Behandlungsvorrichtung (200), und mit wenigstens einer zweiten Haltevorrichtung (102), wobei das Modul (301) mit der ersten Haltevorrichtung (101) und mit der zweiten Haltevorrichtung (102) lösbar verbunden ist, und wobei die erste Haltevorrichtung (101) und die zweite Haltevorrichtung (102) jeweils wenigstens eine erste Aufnahme (101a, 102a) und jeweils eine zweite Aufnahme (101b, 102b) aufweisen, und wobei die zweite Haltevorrichtung (102) sowohl mit dem Modul (301) als auch mit wenigstens einem weiteren Modul (302) oder mit einem Adapter zur Aufnahme eines weiteren Moduls (302) verbunden ist.

2. Anordnung nach Anspruch 1, wobei die erste Aufnahme (101a, 102a) ein Klemmhalter mit Klemmbacken (101a1, 101a2) ist oder einen solchen Klemmhalter aufweist, wobei die Klemmbacken (101a1, 101a2) relativ zueinander bewegbar und/oder von einer ersten Stellung zueinander in eine zweite Stellung zueinander bewegbar angeordnet sind.

3. Anordnung nach Anspruch 1 oder 2, wobei die zweite Aufnahme (101b) der ersten Haltevorrichtung (101) ein Befestigungsabschnitt ist, oder einen solchen aufweist, zum Fixieren der ersten Haltevorrichtung (101) an einem Abschnitt der medizinischen Behandlungsvorrichtung (200).

4. Anordnung nach einem der vorangegangenen Ansprüche, wobei die erste und/oder die zweite Haltevorrichtung (101, 102) zusätzlich zur ersten Aufnahme (102a) und zur zweiten Aufnahme (102b) eine dritte Aufnahme (102c) aufweist.

5. Anordnung nach einem der vorangegangenen Ansprüche, wobei wenigstens zwei Elemente aus der Gruppe, welche aus der ersten Aufnahme (101a, 102a), der zweiten Aufnahme (101b, 102b) und der dritten Aufnahme (102c) besteht, ein Klemmhalter mit Klemmbacken (101a1, 101a2) ist oder aufweist, wobei die Klemmbacken (101a1, 101a2) relativ zueinander bewegbar und/oder von einer ersten Stellung zueinander in eine zweite Stellung zueinander bewegbar angeordnet sind.

6. Anordnung nach einem der vorangegangenen Ansprüche, wobei zwischen der ersten Aufnahme (101a, 102a) und der zweiten Aufnahme (101b, 102b) und/oder zwischen der ersten Aufnahme (102a) und der dritten Aufnahme (102c) und/oder zwischen der zweiten Aufnahme (102b) und der dritten Aufnahme (102c) ein Drehabschnitt (102d) angeordnet ist, welcher eine Drehbewegung der ersten Aufnahme (101a, 102a) relativ zur zweiten Aufnahme (101b, 102b) ermöglicht.

7. Anordnung nach einem der vorangegangenen Ansprüche, wobei die erste Aufnahme (101a, 102a) und die zweite Aufnahme (101b, 102b) identisch ausgestaltet sind, und/oder die erste Aufnahme (102a) und die dritte Aufnahme (102c) identisch ausgestaltet sind und/oder die zweite Aufnahme (102b) und die dritte Aufnahme (102c) identisch ausgestaltet sind und/oder die erste Aufnahme (102a), die zweite Aufnahme (102b) und die dritte Aufnahme (102c) identisch ausgestaltet sind.

8. Anordnung nach einem der Ansprüche 4 bis 7, wobei durch die erste Aufnahme (102a) und durch die dritte Aufnahme (102c) eine Gerade verläuft, welche vorzugsweise nicht auch durch die zweite Aufnahme (102b) verläuft, oder welche vorzugsweise auch durch die zweite Aufnahme (102b) verläuft.

9. Anordnung nach einem der Ansprüche 4 bis 8, wobei ein Abstand zwischen der ersten Aufnahme (102a) und der dritten Aufnahme (102c), vorzugweise entlang der Geraden, wenigstens so groß ist wie eine Breite der ersten Aufnahme (102a), vorzugsweise in einer Richtung entlang der Geraden.

10. Anordnung nach einem der vorangegangenen Ansprüche, wobei die erste und/oder zweite Haltevorrichtung (101, 102) eine Stützstruktur (102e) zum Abstützen der ersten und/oder zweiten Haltevorrichtung (101, 102) aufweist.

11. Anordnung nach Anspruch 10, wobei die erste Aufnahme (101a, 102a) zwischen der Stützstruktur (102e) und der zweiten Aufnahme (101b, 102b) angeordnet ist, und/oder wobei die Stützstruktur (102e) an der ersten Aufnahme (101a, 102a) und/oder an der zweiten Aufnahme (101b, 102b) angeordnet ist.

12. Anordnung nach einem der vorangegangenen Ansprüche, wobei die zweite Haltevorrichtung (102) sowohl oberhalb als auch unterhalb der ersten Aufnahme (101a) der ersten Haltevorrichtung (101) mit dem Modul (301) verbunden ist.

13. Anordnung nach einem der vorangegangenen Ansprüche, wobei die zweite Haltevorrichtung (102) angeordnet ist, um sich mit einer Stützstruktur (102e) hiervon gegen die erste Haltevorrichtung (101) abzustützen.

14. Medizinische Behandlungsvorrichtung (200), die mittels der ersten Haltevorrichtung (101) mit der Anordnung gemäß einem der Ansprüche 1 bis 13 verbunden ist.

15. Medizinische Behandlungsvorrichtung (200) nach Anspruch 14, welche mittels der ersten Haltevorrichtung (101) mit dem medizinischen Modul (301) verbunden ist, wobei das Modul (301) mit wenigstens einer zweiten Haltevorrichtung (102) verbunden ist, wobei die zweite Haltevorrichtung (102) mit einem zweiten Modul (302) oder einem Adapter zum Aufnehmen eines zweiten Moduls (302) verbunden ist, wobei das zweite Modul (302) oder der Adapter mit einer dritten Haltevorrichtung (103) verbunden ist.

## Claims

1. An arrangement comprising a medical module (301) designed as a filter, a blood filter, a dialyzer, a cartridge, a gas exchanger or an adsorber, comprising at least one first holder (101) for releasably holding the module (301) to a medical treatment apparatus (200), and comprising at least one second holder (102), wherein the module (301) is releasably connected with the first holder (101) and with the second holder (102), and wherein the first holder (101) and the second holder (102) each comprise at least one first reception unit (101a, 102a) and each comprise a second reception unit (101b, 102b), and wherein the second holder (102) is connected both with the module (301) and with at least one further module (302) or with an adapter for receiving a further module (302).

2. The arrangement according to claim 1, wherein the first reception unit (101a, 102 a) is or comprises a clamping holder having clamp jaws (101a1, 101a2), wherein the clamp jaws (101a1, 101a2) are arranged movable relative to each other and/or movable from a first position to each other into a second position to each other.

3. The arrangement according to claim 1 or 2, wherein the second reception unit (101b) of the first holder (101) is, or comprises, a fixing section for fixing the first holder (101) on a section of the medical treatment apparatus (200).

4. The arrangement according to anyone of the preceding claims, wherein the first and/or the second holder (101, 102) comprise (s) a third reception unit (102c) in addition to the first reception unit (102a) and the second reception unit (102b).

5. The arrangement according to anyone of the preceding claims, wherein at least two elements from the group consisting of the first reception unit (101a, 102a), the second reception unit (101b, 102b) and the third reception unit (102c) are or comprise a clamping holder with clamp jaws (101a1, 101a2), wherein the clamp jaws (101a1, 101a2) are arranged movable relative to each other and/or movable from a first position to each other into a second position to each other.

6. The arrangement according to anyone of the preceding claims, wherein a rotating section (102d) is arranged between the first reception unit (101a, 102a) and the second reception unit (101b, 102b) and/or between the first reception unit (102a) and the third reception unit (102c) and/or between the second reception unit (102b) and the third reception unit (102c), which enables a rotation of the first reception unit (101a, 102a) relative to the second reception unit (101b, 102b).

7. The arrangement according to anyone of the preceding claims, wherein the first reception unit (101a, 102a) and the second reception unit (101b, 102b) are identically embodied and/or the first reception unit (102a) and the third reception unit (102c) are identically embodied and/or the second reception unit (102b) and the third reception unit (102c) are identically embodied and/or the first reception unit (102a), the second reception unit (102b) and the third reception unit (102c) are identically embodied.

8. The arrangement according to anyone of claims 4 to 7, wherein a straight line runs through the first reception unit (102a) and through the third reception unit (102c), which preferably does not also run through the second reception unit (102b), or which preferably also runs through the second reception unit (102b).

9. The arrangement according to anyone of claims 4 to 8, wherein a distance between the first reception unit (102b) and the third reception unit (102c), preferably along the straight line, is at least as large as a width of the first reception unit (102a), preferably in a direction along the straight line.

10. The arrangement according to anyone of the preceding claims, wherein the first and/or the second holder (101, 102) comprise(s) a support structure (102e) for supporting the first and/or second holder (101, 102).

11. The arrangement according claim 10, wherein the first reception unit (101a, 102a) is arranged between the support structure (102e) and the second reception unit (101b, 102b), and/or wherein the support structure (102e) is arranged at the first reception unit (101a, 102a) and/or at the second reception unit (101b, 102b).

12. The arrangement according to anyone of the preceding claims, wherein the second holder (102) is connected to the module (301) both above and below the first reception unit (101a) of the first holder (101).

13. The arrangement according to anyone of the preceding claims, wherein the second holder (102) is arranged to brace against the first holder (101) by means of a support structure (102e) thereof.

14. A medical treatment apparatus (200), which is connected with the arrangement, according to anyone of claims 1 to 13, by means of the first holder (101).

15. The medical treatment apparatus (200) according to claim 14, which is connected to the medical module (301) by means of the first holder (101), wherein the module (301) is connected with at least one second holder (102), wherein the second holder (102) is connected with a second module (302) or with an adapter for receiving a second module (302), wherein the second module (302) or the adapter is connected with a third holder (103).

## Revendications

1. Un agencement présentant un module médical (301) conçu comme un filtre, un filtre sanguin, un dialyseur, une cartouche, un échangeur de gaz ou un adsorbeur, comprenant au moins un premier support (101) pour maintenir le module (301) de manière amovible sur un appareil de traitement médical (200), et comprenant au moins un second support (102),
où le module (301) est relié de manière amovible au premier support (101) ainsi qu'au second support (102),
et où le premier support (101) et le second support (102) comprennent chacun au moins une première unité de réception (101a, 102a) et comprennent chacun une seconde unité de réception (101b, 102b), et où le second support (102) est relié aussi bien au module (301) qu'à au moins un autre module (302) ou qu'à un adaptateur destiné à recevoir un autre module (302).

2. L'agencement selon la première revendication, où la première unité de réception (101a, 102 a) est ou comprend un support de serrage ayant des mâchoires de serrage (101a1, 101a2), où les mâchoires de serrage (101a1, 101a2) sont agencées de manière à être mobiles l'une par rapport à l'autre et/ou de manière à être mobiles d'une première position l'une par rapport à l'autre à une seconde position l'une par rapport à l'autre.

3. L'agencement selon la revendication 1 ou 2, où la seconde unité de réception (101b) du premier support (101) est, ou comprend, une section de fixation pour fixer le premier support (101) à une section de l'appareil de traitement médical (200).

4. L'agencement selon l'une quelconque des revendications précédentes, où le premier et/ou le second support (101, 102) comprend(ennent) une troisième unité de réception (102c) en plus de la première unité de réception (102a) et de la seconde unité de réception (102b).

5. L'agencement selon l'une quelconque des revendications précédentes, où au moins deux éléments du groupe constitués par la première unité de réception (101a, 102a), la seconde unité de réception (101b, 102b) ainsi que la troisième unité de réception (102c), sont, ou comprennent, un support de serrage avec des mâchoires de serrage (101a1, 101a2), où les mâchoires de serrage (101a1, 101a2) sont agencées de manière à être mobiles l'une par rapport à l'autre et/ou de manière à être mobiles d'une première position l'une par rapport à l'autre à une seconde position l'une par rapport à l'autre.

6. L'agencement selon l'une quelconque des revendications précédentes, où une section rotative (102d) est agencée entre la première unité de réception (101a, 102a) et la seconde unité de réception (101b, 102b) et/ou entre la première unité de réception (102a) et la troisième unité de réception (102c) et/ou entre la seconde unité de réception (102b) et la troisième unité de réception (102c), laquelle permet une rotation de la première unité de réception (101a, 102a) par rapport à la seconde unité de réception (101b, 102b).

7. L'agencement selon l'une quelconque des revendications précédentes, où la première unité de réception (101a, 102a) et la seconde unité de réception (101b, 102b) sont conçues de manière identique et/ou la première unité de réception (102a) et la troisième unité de réception (102c) sont conçues de manière identique et/ou la seconde unité de réception (102b) et la troisième unité de réception (102c) sont conçues de manière identique et/ou la première unité de réception (102a), la seconde unité de réception (102b) et la troisième unité de réception (102c) sont conçues de manière identique.

8. L'agencement selon l'une quelconque des revendications 4 à 7, où une ligne droite passe par la première unité de réception (102a) et par la troisième unité de réception (102c), qui, de préférence, ne passe pas par la seconde unité de réception (102b), ou qui, de préférence, passe également par la seconde unité de réception (102b).

9. L'agencement selon l'une quelconque des revendications 4 à 8, où une distance entre la première unité de réception (102b) et la troisième unité de réception (102c), de préférence longeant la ligne droite, est au moins aussi grande qu'une largeur de la première unité de réception (102a), de préférence dans une direction longeant la ligne droite.

10. L'agencement selon l'une quelconque des revendications précédentes, où le premier et/ou le second support (101, 102) comprend(ennent) une structure de soutien (102e) pour soutenir le premier et/ou le second support (101, 102).

11. L'agencement selon la revendication 10, où la première unité de réception (101a, 102a) est agencée entre la structure de soutien (102e) et la seconde unité de réception (101b, 102b), et/ou où la structure de soutien (102e) est agencée sur la première unité de réception (101a, 102a) et/ou sur la seconde unité de réception (101b, 102b).

12. L'agencement selon l'une quelconque des revendications précédentes, où le second support (102) est relié au module (301) à la fois au-dessus et au-dessous de la première unité de réception (101a) du premier support (101).

13. L'agencement selon l'une quelconque des revendications précédentes, où le second support (102) est agencé de façon à s'appuyer contre le premier support (101) via sa structure de soutien (102e).

14. Un appareil de traitement médical (200) qui est relié à l'agencement, selon l'une quelconque des revendications 1 à 13, au moyen du premier support (101).

15. L'appareil de traitement médical (200) selon la revendication 14, qui est relié au module médical (301) au moyen du premier support (101), où le module (301) est relié au moins à un second support (102), où le second support (102) est relié à un second module (302) ou à un adaptateur pour la réception d'un second module (302), où le second module (302) ou l'adaptateur est relié à un troisième support (103).
